# EUROPEAN PATENT SPECIFICATION

(11) **EP 1 412 471 B1**
(45) Date of publication and mention of the grant of the patent: **06.10.2010**
(21) Application number: 01959345.8
(22) Date of filing: 31.07.2001
(51) Int. Cl.: C07K 16/40

(54) **APPARATUS AND METHOD FOR DETERMINING THE ONSET AND PRESENCE OF SEPSIS CONDITIONS**
VORRICHTUNG UND VERFAHREN ZUR BESTIMMUNG DES EINTRETENS UND VORHANDENSEINS SEPTISCHER BEDINGUNGEN
DISPOSITIF ET PROCEDE PERMETTANT DE DETECTER L'APPARITION ET LA PRESENCE DE CONDITIONS DE SEPSIE

(30) Priority: 31.07.2000 US 628585
(43) Date of publication of application: 28.04.2004
(73) Proprietor: WEBBER, Robert, Benicia, CA 94510 (US)
(72) Inventor: WEBBER, Robert, Benicia, CA 94510 (US)
(74) Representative: Modiano, Micaela Nadia
(86) International application number: PCT/US2001/023943
(87) International publication number: WO 2003/012026

(56) References cited:
- WO-A1-96/39858
- WO-A1-98/45710
- US-A- 5 206 161
- ANONYMOUS: "Monoclonal antibody based immunoassays for human inducible nitric oxide synthase as indicators of sepsis and septic shock" FASEB JOURNAL, vol. 11, no. 3, 1997, page A662, XP008079547 & ANNUAL MEETING OF THE PROFESSIONAL RESEARCH SCIENTISTS ON EXPERIMENTAL BIOLOGY 97; NEW ORLEANS, LOUISIANA, USA; APRIL 6-9, 1997 ISSN: 0892-6638
- WEBBER ROBERT ET AL: "Can the expression of iNOS (NOS type 2) in circulating leukocytes be used to predict severe sepsis/septic shock?" NITRIC OXIDE, vol. 4, no. 3, 2000, pages 293-294, XP008079535 & FIRST INTERNATIONAL CONFERENCE ON BIOLOGY, CHEMISTRY, AND THERAPEUTIC APPLICATIONS OF NITRIC OXIDE; SAN FRANCISCO, CALIFORNIA, USA; JUNE 03-07, 2000 ISSN: 1089-8603
- MCCAPRA F. ET AL: 'Luminescent labels for immunoassay - from concept to practice' JOURNAL OF BIOLUMINESCENCE AND CHEMILUMINESCENCE vol. 4, no. 1, July 1989, pages 51 - 58

## Description

### BACKGROUND OF THE INVENTION

The present invention relates to the use of an apparatus and method for rapidly determining the onset and presence of a sepsis condition.

Infections in a mammalian subject such as a human can occur directly by different types of organisms and indirectly through medical trauma. For example, gram-negative and gram-positive bacteria, viruses, and fungi may cause such infections. In certain cases, "sepsis conditions" may result from such infection. Such "sepsis conditions" include systemic inflammatory response syndrome (SIRS), sepsis, severe sepsis, and septic shock. The latter condition causes multiple organ failure in a subject. It is estimated that hundreds of thousands of deaths occur each year due to septic shock in the United States alone.

An early prognostication of a sepsis condition is extremely important in order to initiate therapies to treat patients having this malady. For example, current therapies for the treatment of sepsis conditions include the administering of combined antibiotics and fluid replacement. It is known that the initial systemic inflammatory response syndrome (SIRS) is defined as a condition in which two or more of the following are present in a human:
1. Temperature less than 36°C or greater than 38°C;
2. A heart rate of greater than 90;
3. A respiratory rate of greater than 20; or
4. A white blood cell count of less than 4 x 10⁶/ml or greater than 12 x 10⁶/ml. "Sepsis" is a condition of SIR plus a culture-documented infection. [Sepsis patients possess an increase in cardiac output, such as that found in systemic vascular resistance diseases, but with low organ perfusion.] "Severe sepsis" is a condition of sepsis with the addition of organ dysfunction, hypotension, or hypoperfusion. "Septic shock" is defined as hypotension (despite fluid resuscitation) plus hypoperfusion abnormalities. Organ failure occurs as a result of cell death due to tissue hypoperfusion.

Causative agents of sepsis conditions above described point to the overproduction of nitric oxide (NO) by the inducible form of nitric oxide synthase (iNOS). It has been demonstrated that the lipopolysaccharide (LPS) component from the cell wall of a gram-negative bacteria and the combination of lipoteichoic acid and peptidoglycan from the cell wall of a gram-positive bacteria result in the release of inflammatory cytokines such as TNFα, IL-1β, and IL-6. The interaction of the bacterial components and the inflammatory cytokines, in turn can induce the production of iNOS. The increased formation of NO generated by the iNOS then leads to increased vascular permeability, vasodilatation, hypotension, tissue hypoperfusion, and ultimately organ failure. As discussed above, these states are the manifestations of severe sepsis and septic shock. Thus, it is generally believed that it is the action of NO on smooth muscles and the vascular endothelium that results in systemic vasodilatation and increased vascular permeability. This, in turn, is responsible for the hypotension and tissue hypoperfusion resulting in multiple organ failure.

Although the therapies used to treat sepsis conditions are useful, meaningful prognosticators are needed to allow for the earliest possible intervention. It has been noted in an article entitled "Gram-Negative Bacteremia, IV: Reevaluation of Clinical Features in Treatment in 612 Patients" by Kreger et al that the early initiation of antibiotics reduce the frequency of septic shock and death by 50 percent.

At present, a definitive rapid clinical diagnostic for sepsis conditions such as SIRS and sepsis does not exist. Among the current procedures is one described in an article entitled "Epidemiology of Sepsis Syndrome in 8 Academic Medical Centers" by Sands et al includes blood culture procedures. Unfortunately blood culture techniques are slow, taking 24 to 48 hours for a result. In addition, blood culture tests are inaccurate, only yielding positive results indicating the presence of a sepsis condition in approximately 28 percent of patients with SIRS.

An article entitled "Assessment of the Safety and Efficacy of the Monoclonal Anti-Tumor Necrosis Factor Antibody-Fragment, MAK 195F, in Patients with Sepsis and Septic Shock: A Multicenter, Randomized, Placebo-Controlled, Dose-Ranging Study" by Rinheart et al, and another article entitled "Interleukin-6 Measurements in Selection of Sepsis Patients for Anti-cytokine Therapy", by Fischkoff indicate that the presence of interleukin-6 (IL-6) greater than 1,000 pg/ml is associated with hyperinflammatory conditions and is only predictive of poor outcome in septic patients receiving monoclonal treatment, MAK 195F.

United States Patent 5,639,617 describes the early detection of a sepsis in a patient by the recognition of the presence of procalcitonin in a biological liquid of the patient. However, the existence of procalcitonin only indicates a single source of the sepsis condition i.e. gram-negative bacteria.

Increases in the concentration of nitrate or nitrite in the blood has also been linked to sepsis. However, the presence of nitrate/nitrite in the blood has not been positively correlated to the status of patients with SIRS or sepsis conditions.

The detection of iNOS, in sepsis patients from induced leukocytes of sepsis patients has been accomplished. However such determination reveals a highly variable amount of iNOS and is not necessarily correlated to the presence of a sepsis condition.

An assay for rapidly determining the onset and presence of SIRS, sepsis, severe sepsis, and/or septic shock, would be a notable advance in the medical field. WO 9845710 A discloses detection of septic shock using an immunoassay for detecting iNOS without cross-reacting with eNOS or nNOS in cell lysates from whole blood.

### BRIEF SUMMARY OF THE INVENTION

In accordance with the present invention a novel and useful method for performing an assay to determine the presence of sepsis conditions is herein provided.

The immunoassay of the present invention is especially useful for measuring the level of iNOS in the liquid portion of blood selected from plasma or serum.

It has been found that the intracellular enzyme iNOS is released in small quantities into the circulatory system of mammalian subjects, such as human patients, 24 to 48 hours prior to the onset of severe sepsis, during the early stages of SIRS. Thus, the detection and measurement of iNOS in the liquid portion of blood selected from plasma or serum by the immunoassay of the present invention serves as a useful prognosticator for the onset of sepsis conditions such as SIRS, sepsis, severe sepsis, and septic shock. It has also been found that the quantitative measurement of iNOS in plasma, serum, and whole blood can be used to monitor the course of a sepsis condition and to assesses the course of treatment of a sepsis condition.

The present invention involves a chemiluminescent sandwich immunoassay (EIA or Elisa) for use in detecting and measuring the concentration of inducible nitric oxide sythase (iNOS) in the blood of a mammalian subject as defined in claim 1. Specifically, the liquid portion of the blood is employed in this regard. One aspect of the invention involves the use of a sandwich solid phase immunoassay which employs two sites. The first site uses an anti-iNOS monoclonal antibody that may be anti-iNOS clone identified as 21C10-1D10, which is found in United States Patent Application Serial Number 08/833,506 filed 7 April 1997. Such monoclonal antibody is known as the "capture" antibody, such that it binds any iNOS contained in a sample of the liquid portion of the blood of the mammalian subject. A labeled second monoclonal antibody is also employed as the "detection" antibody in a one step or two step reaction sequence. For example, biotinylated anti-iNOS monoclonal antibody identified as clone 2A1-F8 in the above-identified patent application suffices in this regard. The assay takes place on a solid support or carrier which can be any solid material capable of binding iNOS or anti-iNOS antibodies. Such solid support is well known is immunoassays and includes, but is not limited to, polystyrene, polypropylene, polyethylene, polyacrylamides, agarose, glass, dextrans, nylon, magnetite, cellulose, modified cellulose, and amylases. The support may be spherical, as a bead, or flat such as a sheet or test strip. In addition, the support may be cylindrical, akin to the inner surface of a test tube. In essence, the solid support or carrier may have virtually any structural configuration as long as the capture antibody and the iNOS can bond together thereupon. For example, a micro titer plate well has been found to perform this function successfully.

In one form of the invention, the labeled detection antibody is bound to a "binding partner" such as streptavidin, avidin, avidin-biotin complex (ABC), an anti-biotin antibody or the like. The binding partner is, in turn, conjugated to an enzyme such as horseradish peroxidase in an enzyme immunoassay (EIA) or enzyme-linked immunosorbent assay (ELISA) format.

Alternatively, the purified detection antibody or its binding partner can be directly conjugated to an enzyme or luminescent compounds

In any case, enzymes that may be used as labels for conjugation to the purified detection antibody are chosen to produce luminescent products which can be detected by spectrophotometric, fluorometric, luminescent, visual or other means. Enzyme labels which are useful in EIAs and ELISAs include, but are not limited to, horseradish peroxidase, alkaline phosphatase, glucose oxidase, glucose-6-phosphate dehydrogenase, catalase, beta-galactosidase, glucoamylase, acetylcholinesterase, malate dehydrogenase, urease, alcohol dehydrogenase, ribonuclease, asparaginase, staphylococcal nuclease, triose phosphate isomerase, delta-5-steroid isomerase, and alpha-glycerophosphate dehydrogenase.

The detection antibody may be labeled directly to the fluorochrome luminescent compounds and the like. In the case of an indirect reaction sequence, the labeled detection antibody is bound to an appropriately binding partner, and unbound material is removed by washing. Chemiluminescent substrates, for example, may be added such that the bound enzyme converts the substrate into products which emit photons of light that can be detected and measured by a luminometer. Consequently, the amount of light that is produced during a fixed length of time is directly proportional to the amount of iNOS contained in the liquid portion of the blood serving as the sample or specimen.

Luminescent compounds which are useful in EIA or the ELISA formats include, but are not limited to, luminol, isoluminol, theromatic acridinium ester, oxalate ester, acridinium salts, imidazole, and the like.

Luminescent enzymes which release photons of light during catalysis conjugated to the detection antibody or its binding partner useful in EIA or ELISA formats, include, but are not limited to, luciferase, luciferan, and aequorin.

Other test formats may be employed such as dipsticks, lateral flow devices, auto-analyzers, point-of-care devices, and the like. In any case, the developments of SIRS, sepsis, severe sepsis, or septic shock may be detected prior to onset or during occurrence of such sepsis conditions, as well as during a treatment phase following occurrence.

It may be understood a useful apparatus and method for the detection of SIRS, sepsis, severe sepsis, or septic shock has been described.

It is therefore an object of the present invention to provide an method and use of an assay apparatus to detect a sepsis condition which employs the liquid portion of the blood selected from plasma or serum of a mammalian subject.

Another object of the present invention is to provide a use of an apparatus and method for detecting the onset and/or existence of a sepsis condition which is relatively quick and permits rapid therapeutic treatment of patients.

A further object of the present invention is to provide a use of an apparatus and method for detecting the onset and/or presence of a sepsis condition in a mammalian subject which is accurate.

A further object of the present invention is to provide a method and use of an apparatus for detecting the onset and/or presence of a sepsis condition which is capable of monitoring therapies applied to patients detected as having a sepsis condition.

Another object of the present invention is to provide a use of an apparatus and method for detecting the onset and/or presence of a septic condition in a mammalian subject which permits the application of therapies to a patient at an early stage, thus increasing the probability of survival of a patient and reducing the length of treatment in an intensive care unit.

The invention possesses other objects and advantages especially as concerns particular characteristics and features thereof which will become apparent as the specification continues.

### BRIEF DESCRIPTION OF THE SEVERAL VIEWS OF THE DRAWING

Fig. 1 is a graph illustrating the purification of the IgG fraction of anti-hiNOS monoclonal antibodies described in Example 1.
Fig. 2 is a graph showing a relation between chemiluminescence and iNOS detected in Example 2.
Fig. 3A is a graphical representation of a patient's iNOS level during the onset phase of SIRS/sepsis, as described in Example 3.
Fig. 3B is a graphical representation of a patient's iNOS level following treatment for sepsis, as described in Example 3.
Fig. 4 is a reproduction of a western blot analysis of plasma samples of individuals with SIRS and/or sepsis confirming the presence of iNOS, described in Example 3.
Fig. 5 is a graph representing the non-correlation of the concentration of nitrite or nitrate in plasma to the presence of iNOS in the plasma of patients with SIRS and/or sepsis as described in Example 4.
Fig. 6 is a graph representing the concentration of iNOS in plasma between a group of patients with SIRS or sepsis compared to healthy volunteers, as described in Example 5.

For a better understanding of the invention reference is made to the following detailed description of the preferred embodiments and examples which should be taken in conjunction with the herein above described drawings.

### DETAILED DESCRIPTION OF THE PREFERRED EMBODIMENTS OF THE

### INVENTION

Various aspects of the invention will evolve from the following detailed description of the preferred embodiments and examples thereof which should be viewed in conjunction with the prior described drawings.

The apparatus and method of the present invention rapidly determines the onset or presence of systemic inflammatory response syndrome (SIRS), sepsis, severe sepsis, or septic shock based on analysis performed on the liquid portion of the blood selected from plasma or serum of a mammalian subject such as a human patient. The apparatus includes means for detecting the existence of IC inducible nitric oxide synthase (iNOS) in the liquid portion of the blood selected from plasma or serum of the human patient or mammalian subject without also detecting, or cross reacting, with eNOS or nNOS. Specifically, such means may include an immunoassay that can rapidly detect and quantitate the presence or change in amounts of iNOS in plasma or serum. It has been found that iNOS is generated by a human patient early in an episode of SIRS and prior to the onset of severe sepsis and septic shock.

The assay of the present invention utilizes a monoclonal antibody, preferably the IgG fraction of anti-iNOS monoclonal antibody clone 21C10-ID10 which binds to residues number 39-45 of human iNOS. Such clone is identified in prior noted patent application serial number 08/833,506. The antibody is adsorbed onto a solid support or carrier, preferably the wells of a white EIA microtiter plate. This antibody is known as the "capture antibody" and is used to bind the iNOS contained in a sample or specimen of the liquid portion of the blood of the mammalian subject. Such liquid sample is plasma or serum. Simultaneously a labeled detection antibody, anti-iNOS monoclonal antibody, is bond to form a sandwich in a one step reaction. Such label detection antibody is preferably a biotinylated anti-iNOS monoclonal antibody as the IgG fraction of clone 21A1-F8 identified in the above-mentioned patent application. Following the formation of the sandwich, unbound labeled detection antibody is removed by washing and an excess amount of an appropriate binding partner, such as enzyme conjugated streptavidin or enzyme conjugated anti-biotin antibody is bound. Again, unbound material is removed by washing and a chemiliuminecent substrate is added. The bound enzyme converts the substrate into certain products with the emission of photons of light. Such photons are detected and measured by a luminometer. The amount of light that is produced during a fixed length of time is directly proportional to the amount of iNOS contained in the sample or specimen of the liquid portion of the blood of the mammalian subject. In other words, as the amount of iNOS increases in the liquid portion of the blood of the mammalian subject, the amount of light generated by the assay of the present invention increases.

The method of the present invention which determines the existence and quantity of iNOS in the liquid portion of the blood of the mammalian subject may be performed rapidly, namely between one and two hours. To aid in the reduction of time necessary to perform the method using the apparatus of the present invention, absorption of the capture antibody onto a solid support is performed in advance. The capture antibody and solid support are then stored until needed.

Where the assay takes the form of a sandwich enzyme immunoassay (EIA), a monoclonal antibody such as the IgG fraction of anti-iNOS monoclonal antibody clone 21C10-1D10 is absorbed onto the wells of a white 96 well-IEA microtiter plate for two hours or longer. The unbound material is removed in a washing step and unreacted sites are blocked with an excess of a non-specific protein such as bovine serum albumin or gelatin. The unbound material is then washed off and the plate is stored until needed. When a measurement of the amount of iNOS contained in a liquid portion of blood of a mammalian subject is to be made, the sample of the same and the IgG fraction of biotinylated anti-iNOS monoclonal antibody clone 2A1-F8 are added to each well and kept there for 60 minutes. During this time, the iNOS in the sample binds to the immobilized capture antibody and the biotin-2A1-F8 binds to the iNOS in the blood sample, in a one step reaction sequence. Alternatively the biotin 2A1-F8 conjugate can be added separately after the iNOS contained in a sample has been bound and the unbound materials are washed off, a two-step reaction sequence. In either the one or two steps reaction sequence, after incubation, the unbound materials are removed by washing and a binding partner such as enzyme labeled streptavidin, and preferably, horseradish peroxide conjugated streptavidin is added and allowed to bind for 20 minutes. Again, unbound material is removed by washing and a chemiluminescent substrate is added. Preferably, the chemiluminescent substrate takes the form of luminol with chemiluminescent enhancer and hydrogen peroxide. Reaction between the immobilized enzyme labeled component and the chemiluminescent material will result in the production of light which is measured in a microtiter plate luminometer. Moreover, the quantity of light is directly proportional to the quantity of iNOS in the sample of the blood portion of the mammalian subject.

The following examples are deemed to be illustrative of the invention but are not intended to limit the scope of the same in any manner.

### EXAMPLE 1

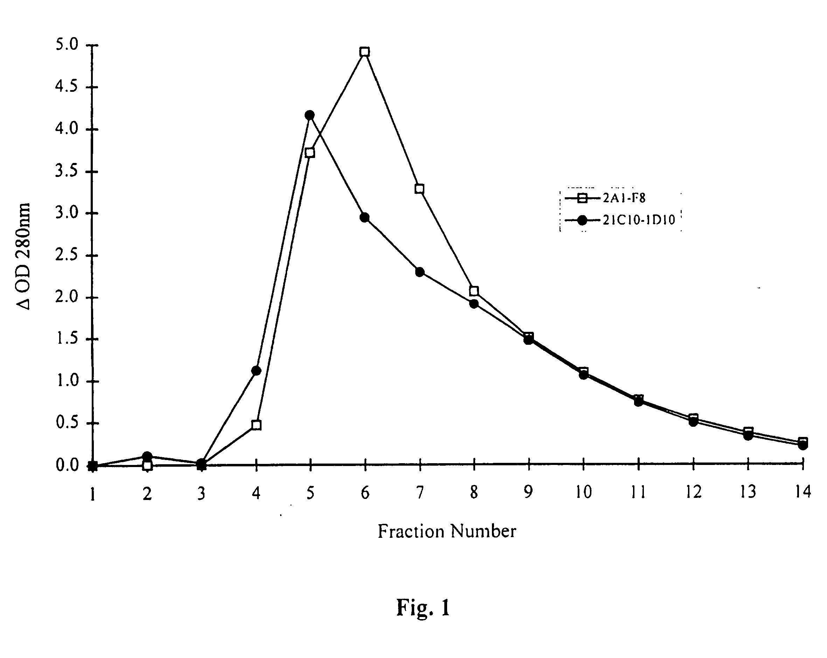

Mouse IgG from two anti-iNOS monoclonal antibody producing clones identified in United States Patent serial number 08/833,506 as clones 21C10-1D10 and 2A1-F8 were obtained. The antibodies were purified either from culture supernatant fluid by protein G affinity purification or from ascites fluid by ammonium sulfate precipitation followed by Sephadex G 200 gel filtration chromatography. Fig. 1 illustrates the gel filtration chromatography results. The purified 21C10-1D10 antibody was absorbed onto microtiter plates for use as the "capture" antibody in a sandwich EIA. 5.0 mg of purified IgG from clone 2A1-F8 was reacted with a ten fold molar excess of sulfosuccinimidyl 6-(biotinamido) hexanoate, obtained from the Pierce Chemical Co. of Rockville, Illinois. A reaction took place in a sodium bicarbonate buffer pH 8.5 for 16 hours and the reaction was stopped with the addition of 0.1 ml of 1.0 molar Tris buffer pH .5. The unbound biotin was removed by dialysis against 0.1 molar Tris buffer, pH 8.5, for 4 hours and against three changes of phosphate buffered saline. The biotin-2A1-F8 conjugate was used as the "detection" antibody in the sandwich EIA with a suitable binding partner.

### EXAMPLE 2

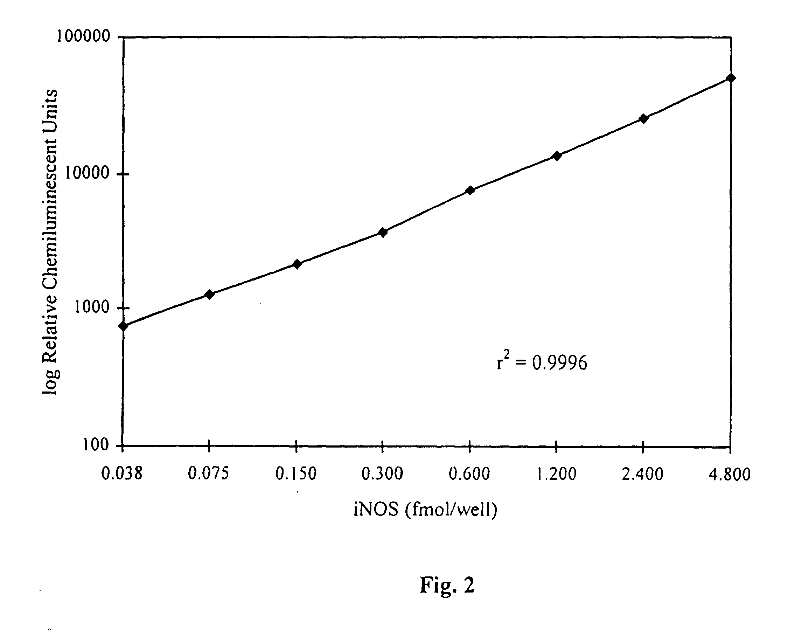

In an attempt to measure iNOS directly in the plasma and/or serum obtained from patients in a clinical study by EIA immunocytochemical staining and western blots, iNOS was found in the non-liquid portion of blood samples, i.e. in induced leukocytes. Free iNOS of the plasma portion was not detected. It is believed that the initial inability to detect iNOS free of plasma was due to the fact that iNOS is an intracellular protein and cells that express iNOS are reported to die by apoptosis. The apoptosis mechanism results in the cell remnants being phagocytosized by macrophages. It was theorized that none of the intracellular components of the leukocytes should be released under this process. It was decided to attempt the detection of iNOS in plasma and/or serum of patients with sepsis by other methods possessing greater sensitivity. Plasma from normal volunteers which should not have contained any iNOS and iNOS purified from induced DLD-1 cells were employed. The latter derived from a human colorectal epithelial adenocarcinoma, which had been reported to express human iNOS after induction with a combination of inflammatory cytokines. The stock solution of purified human iNOS was calibrated by a competitive binding radioimmunoassay using an anti-peptide polyclonal antibody, known amounts of unlabeled free peptide, and ¹²⁵I-labeled peptide. The stock iNOS solution was used to spike normal human plasma with known quantities of iNOS for the development of a highly sensitive EIA. The initial assay employing the EIA of this Example, using ABC as a binding partner with a colorimetric readout, failed to detect iNOS in plasma of SIRS and septic patients. However, substitution of HRP conjugated streptavidin as a binding partner and a chemiluminescent readout resulted in the detection of iNOS spiked into the normal human plasma samples. It is believed that the sensitivity increased 80 fold by this substitution. Fig. 2 represents the results of the chemiluminescent readout. A kit was assembled to perform the immunoassay developed above:
1. One 96 well white EIA microtiter plate coated with the IgG fraction of anti-iNOS clone 21C10-1D10 monoclonal antibody ("capture antibody").
2. Washing buffer.
3. Sample application buffer.
4. Calibrated iNOS standard.
5. Biotinylated IgG fraction of anti-iNOS clone 2A1-F8 monoclonal antibody ("detection antibody").
6. Horseradish peroxide conjugated streptavidin (labeled "binding partner").
7. Chemiluminescent substrates comprised of two components:
   Enhanced chemiluminescent substrate solution (luminol Or isoluminol with enhancer)
   Stabilized hydrogen peroxide solution.
8. Chart/graph for determining the plasma concentration of iNOS.
9. Instruction booklet (operating manual).

The instruction booklet (operating manual), chart/graph, and other data may be provided in computer readable form, such as floppy disk, compact disk, or downloadable Internet file.

### EXAMPLE 3

The sandwich EIA described in Example 2 was standardized for the length of each incubation, buffer composition, and concentration of enzyme label binding partner, for the chemiluminescent readout. iNOS plasma samples were obtained from eight normal healthy volunteers and from ten patients with SIRS/sepsis or patients "at risk" for the development of SIRS/sepsis. Informed consent was obtained from the adults and from the parent or guardian of children prior to enrollment of all volunteers and patients in this study. The eight normal healthy volunteers ranged in age from 18 to 50 years. Three of the healthy volunteers were female and five were male. No iNOS was detected in the plasma obtained from the eight normal healthy volunteers. It was summarized that the level of free iNOS in the plasma of the eight normal healthy volunteers, if existing at all, must be below the normal limit of detection of the assay described in Example 2, i.e. 0.375 fmol/ml.

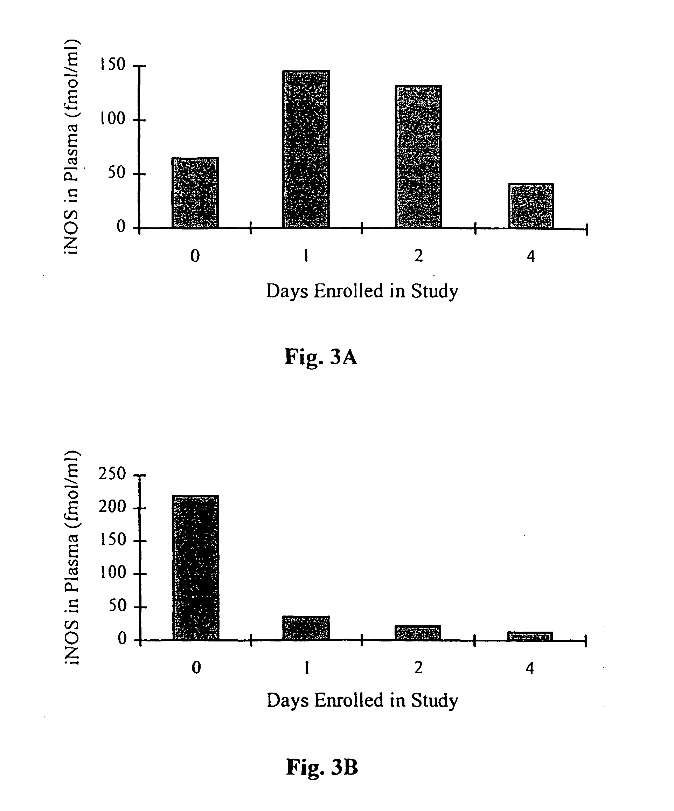

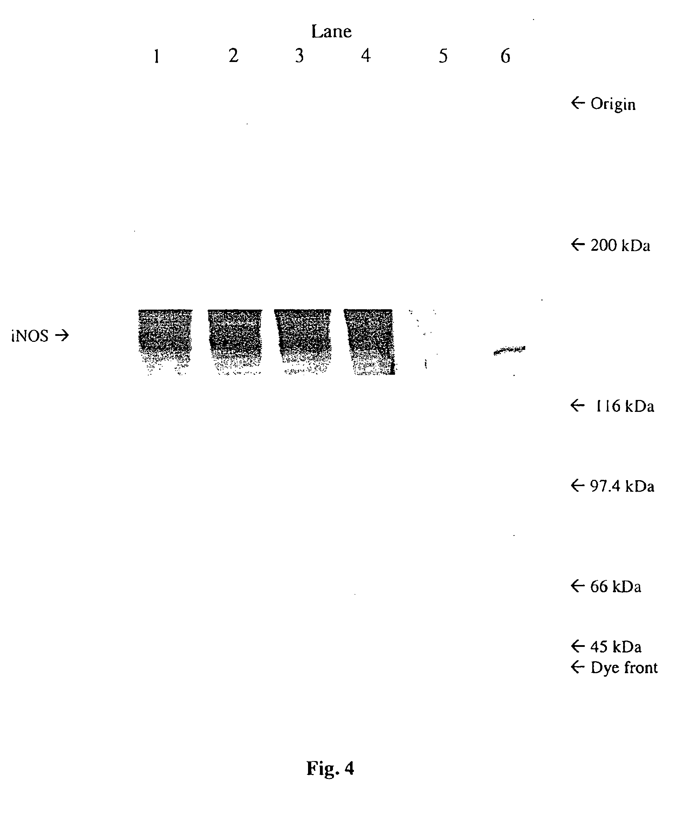

The ten patients with SIRS and sepsis, or "at risk" for developing such pathophysiology were then studied following approved protocol. Three different types of samples were obtained from such patients: Plasma (liquid portion of unclotted blood), serum (the liquid portion of clotted blood), peripheral blood mononuclear cells (PBMCs) fixed on glass slides, and in phosphate buffered saline. Free iNOS was discovered in plasma in the patients with SIRS, sepsis, or "at risk" for developing SIRS or sepsis. Figs. 3A and 3B represent this finding for particular patients in this group. The fact that iNOS is present in the plasma of patients with SIRS, sepsis, and at risk for developing SIRS or sepsis, and not present in the plasma of normal healthy individuals was confirmed by western blot analysis of plasma samples for the presence of iNOS using monoclonal antibody specific for human iNOS. Fig. 4 represents this result.

With reference to Fig. 3A, iNOS from the plasma of a particular patient at risk for the development of SIRS or sepsis was detected 24 to 48 hours prior to the onset of the clinical symptoms of SIRS/sepsis. As may be seen from Fig. 3A, this "at risk" patient possessed elevated plasma iNOS levels 24 hours prior to becoming SIRS positive on day one and 48 hours prior to becoming septic positive on day two. A total of ten ICU patents were tested. One other patient displayed the same pattern of elevated iNOS in plasma 24 to 48 hours prior to the onset of the clinical symptoms of SIRS and sepsis as that shown in Fig. 3A. Moreover, three patients with SIRS and/or sepsis, exhibited a decrease in the level of iNOS in plasma as effective therapy was applied and the patients' condition improved. Fig. 3B shows an example of one of these patients. Two other patients with confirmed sepsis of the ten ICU patients also showed a decreasing level of plasma iNOS as effective treatment was applied, improving the patients' condition.

### EXAMPLE 4

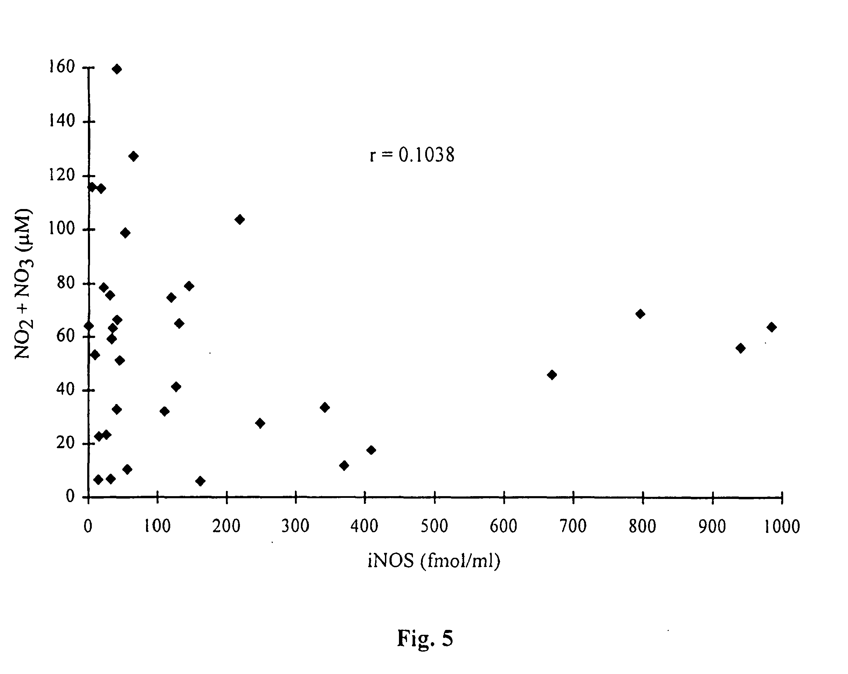

The data obtained for the plasma concentration of iNOS in patients with SIRS and/or sepsis or "at risk" for developing SIRS or sepsis of Example 3 was compared to the plasma concentration of nitrite (NO₂) and nitrate (NO₃). Fig. 5 indicates that there was no statistical significant correlation between the existence of nitrite or nitrate in plasma from these patients. The regression line for this data is defined by y=0.014x + 60.27 and the correlation coefficient r = 0.1038.

### EXAMPLE 5

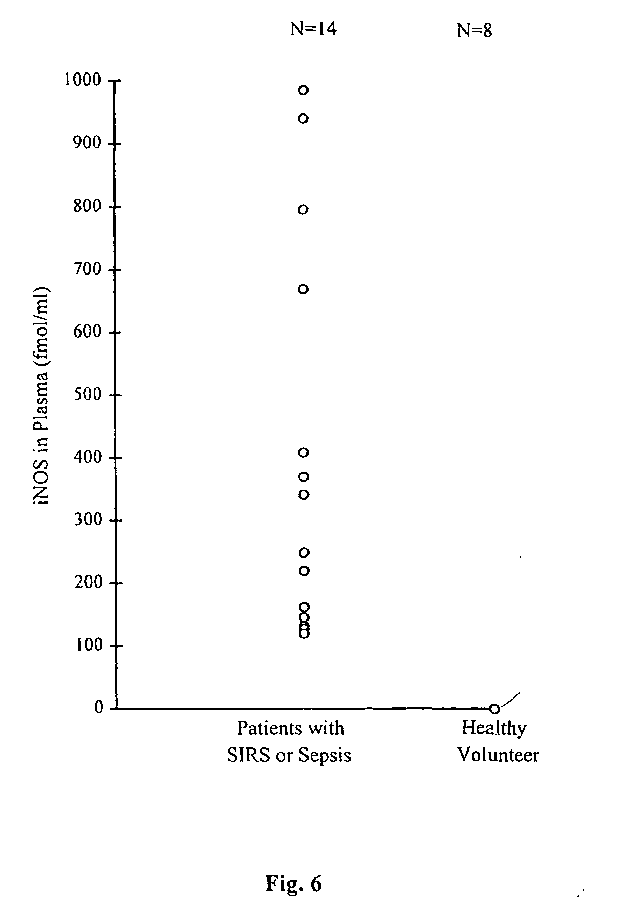

The concentration of iNOS in the plasma, as determined by the chemiluminescent EIA of Example 2, from the 14 samples that were obtained from ten patients with SIRS and/or sepsis prior to treatment, were compared to the concentration found in the plasma of the eight healthy normal volunteers, Fig. 6 shows such results. The data was statistically analyzed by Student's t-Test. The average concentration for the 14 samples from SIRS and septic patients was 404 ± 212 fmol/ml (mean ± standard deviation) and the average for the eight control healthy normal volunteers was zero. By the Students t-Test, the probability that these two populations are the same is less than 0.001 (p < 0.001). Thus, the differences of iNOS measured in the plasma between normal healthy volunteers and patients with SIRS and/or sepsis prior to treatment is highly statistically significant.

## Claims

1. A method for rapidly determining the onset of systemic inflammatory response syndrome, sepsis, severe sepsis, or septic shock in a mammalian subject, said method comprising the step of determining in a sample of the liquid portion of the blood selected from plasma or serum of a mammalian subject the existence of inducible nitric oxide synthase enzyme (iNOS) apart from the existence of endothelial nitric oxide synthase enzyme (eNOS) or neuronal nitric oxide synthase enzyme (nNOS), wherein said step utilizes means for detecting the existence of inducible nitric oxide synthase enzyme (iNOS) in the liquid portion of the blood selected from plasma or serum of a mammalian subject without cross-reacting with eNOS or nNOS, comprising a capture anti-iNOS monoclonal antibody and a labeled detection anti-iNOS monoclonal antibody, respectively, as capture and detection components, in a chemiluminescent sandwich ELISA or chemiluminescent sandwich EIA.

2. The method of claim 1 in which said capture anti-iNOS monoclonal antibody binds to a first site on the inducible nitric oxide synthase (iNOS) in the liquid portion of the blood of a mammalian subject, and said detection anti-iNOS monoclonal antibody binds to a second site on the inducible nitric oxide synthase enzyme (iNOS) in the liquid portion of the blood of a mammalian subject

3. The method of claim 2 in which said detection monoclonal antibody is labeled with biotin, or is directly conjugated to a luminescent compound or a luminescent generating enzyme.

4. The method of claim 2 in which said detection monoclonal antibody is labeled with a first binding partner and which further includes:
a. a second binding partner binding to said first binding partner; and
b. a further label bound to said second binding partner.

5. The method of claim 4 in which said further label is a luminescent compound or a luminescent generating enzyme.

6. The method of claim 4 in which said first binding partner is biotin.

7. The method of claim 4 in which said second binding partner is selected from the group consisting essentially of: strepavidin, avidin, an anti-biotin antibody, and an avidin-biotin complex.

8. The method of claim 7 in which said second binding partner is conjugated to a luminescent generating enzyme.

9. Use of an assay apparatus comprising
means for detecting the existence of inducible nitric oxide synthase enzyme (iNOS) in the liquid portion of the blood selected from plasma or serum of a mammalian subject without cross-reacting with endothelial nitric oxide synthase enzyme (eNOS) or neuronal nitric oxide synthase enzyme (nNOS), said means comprising
a. a capture anti-iNOS monoclonal antibody and a labeled detection anti-iNOS monoclonal antibody; and
b. an assay format selected from the group consisting of:
chemiluminescent sandwich ELISA and chemiluminescent sandwich EIA, utilizing said anti-iNOS monoclonal antibodies, respectively, as capture and detection components in said assay,
for rapidly determining the onset of systemic inflammatory response syndrome, sepsis, severe sepsis, or septic shock in a mammalian subject by detecting the existence of inducible nitric oxide synthase enzyme (iNOS) in the liquid portion of the blood selected from plasma or serum of a mammalian subject.

10. The use of claim 9 in which said capture anti-iNOS monoclonal antibody binds to a first site on the inducible nitric oxide synthase (iNOS) in the liquid portion of the blood of a mammalian subject, and said detection anti-iNOS monoclonal antibody binds to a second site on the inducible nitric oxide synthase enzyme (iNOS) in the liquid portion of the blood of a mammalian subject.

11. The use of claim 10 in which said detection monoclonal antibody is labeled with biotin, or is directly conjugated to a luminescent compound or a luminescent generating enzyme.

12. The use of claim 10 in which said detection monoclonal antibody is labeled with a first binding partner and which further includes:
a. a second binding partner binding to said first binding partner; and
b. a further label bound to said second binding partner.

13. The use of claim 12 in which said further label is a luminescent compound or a luminescent generating enzyme.

14. The use of claim 12 in which said first binding partner is biotin.

15. The use of claim 12 in which said second binding partner is selected from the group consisting essentially of: strepavidin, avidin, an anti-biotin antibody, and an avidin-biotin complex.

16. The use of claim 15 in which said second binding partner is conjugated to a luminescent generating enzyme.

## Patentansprüche

1. Ein Verfahren zum Schnellnachweis des Einsetzens des Systemic Inflammatory Response-Syndroms, Sepsis, schwerer Sepsis oder septischen Schocks bei einem Säugetier-Individuum, wobei das Verfahren den Schritt der Bestimmung, in einer Probe des Flüssiganteils des Blutes, gewählt aus Plasma oder Serum eines Säugetier-Individuums, des Vorhandenseins induzierbaren Stickoxid-Synthase-Enzyms (iNOS) ohne das Vorhandensein von Endothel-Stickoxid-Synthase-Enzym (eNOS) oder neuronalem Stickoxid-Synthase-Enzym (nNOS) umfasst, worin der Schritt Mittel zum Nachweis des Vorhandenseins induzierbaren Stickoxid-Synthase-Enzyms (iNOS) im Flüssiganteil des Blutes, gewählt aus Plasma oder Serum eines Säugetier-Individuums, ohne Kreuzreaktion mit eNOS oder nNOS verwendet, umfassend einen monoklonalen Einfang-anti-iNOS-Antikörper beziehungsweise einen markierten monoklonalen Nachweis-anti-iNOS-Antikörper als Einfang- und Nachweiskomponenten in einem Chemilumineszenz-Sandwich-ELISA oder Chemilumineszenz-Sandwich-EIA.

2. Das Verfahren gemäß Anspruch 1, worin sich der monoklonale Einfang-anti-iNOS-Antikörper an eine erste Position an der induzierbaren Stickoxid-Synthase (iNOS) im Flüssiganteil des Blutes eines Säugetier-Individuums bindet und der monoklonale Nachweis-anti-iNOS-Antikörper sich an eine zweite Position am induzierbaren Stickoxid-Synthase-Enzym (iNOS) im Flüssiganteil des Blutes eines Säugetier-Individuums bindet.

3. Das Verfahren gemäß Anspruch 2, worin der monoklonale Nachweis-Antikörper mit Biotin markiert oder direkt mit einer lumineszenten Verbindung oder einem lumineszenten erzeugenden Enzym konjugiert ist.

4. Das Verfahren gemäß Anspruch 2, worin der monoklonale Nachweis-Antikörper mit einem ersten Bindungspartner markiert ist und das weiter Folgendes einschließt:
a. einen zweiten Bindungspartner, der sich an den ersten Bindungspartner bindet, und
b. eine weitere Markierung, die an den zweiten Bindungspartner gebunden ist.

5. Das Verfahren gemäß Anspruch 4, worin die weitere Markierung eine lumineszierende Verbindung oder ein lumineszentes erzeugendes Enzym ist.

6. Das Verfahren gemäß Anspruch 4, worin der erste Bindungspartner Biotin ist.

7. Das Verfahren gemäß Anspruch 4, worin der zweite Bindungspartner gewählt ist aus der Gruppe, die im Wesentlichen aus Streptavidin, Avidin, einem Anti-Biotin-Antikörper und einem Avidin-Biotin-Komplex besteht.

8. Das Verfahren gemäß Anspruch 7, worin der zweite Bindungspartner an ein lumineszentes erzeugendes Enzym konjugiert ist.

9. Verwendung einer Testvorrichtung, die Folgendes umfasst:
Mittel zum Nachweis des Vorhandenseins induzierbaren Stickoxid-Synthase-Enzyms (iNOS) im Flüssiganteil des Blutes, gewählt aus Plasma oder Serum eines Säugetier-Individuums, ohne Kreuzreaktion mit Endothel-Stickoxid-Synthase-Enzym (eNOS) oder neuronalem Stickoxid-Synthase-Enzym (nNOS), wobei die Mittel Folgendes umfassen:
a. einen monoklonalen Einfang-anti-iNOS-Antikörper und einen markierten monoklonalen Nachweis-anti-iNOS-Antikörper; und
b. ein Assay-Format, das gewählt ist aus der Gruppe bestehend aus:
chemilumineszentem Sandwich-ELISA und chemilumineszentem Sandwich-EIA, unter Verwendung der monoklonalen Anti-iNOS-Antikörper als Einfang- beziehungsweise Nachweiskomponenten in dem Assay,
zum Schnellnachweis des Einsetzens des Systemic Inflammatory Response-Syndroms, Sepsis, schwerer Sepsis oder septischen Schocks bei einem Säugetier-Individuum, durch Nachweis des Vorhandenseins induzierbaren Stickoxid-Synthase-Enzyms (iNOS) im Flüssiganteil des Blutes, gewählt aus Plasma oder Serum, eines Säugetier-Individuums.

10. Die Verwendung gemäß Anspruch 9, worin sich der monoklonale Einfang-anti-iNOS-Antikörper an eine erste Position an der induzierbaren Stickoxid-Synthase (iNOS) im Flüssiganteil des Blutes eines Säugetier-Individuums bindet und der monoklonale Nachweis-anti-iNOS-Antikörper sich an eine zweite Position am induzierbaren Stickoxid-Synthase-Enzym (iNOS) im Flüssiganteil des Blutes eines Säugetier-Individuums bindet.

11. Die Verwendung gemäß Anspruch 10, worin der monoklonale Nachweis-Antikörper mit Biotin markiert oder direkt an eine lumineszierende Verbindung oder ein lumineszentes erzeugendes Enzym konjugiert ist.

12. Die Verwendung gemäß Anspruch 10, worin der monoklonale Nachweis-Antikörper mit einem ersten Bindungspartner markiert ist, und die weiter Folgendes einschließt:
a. einen zweiten Bindungspartner, der sich an den ersten Bindungspartner bindet, und
b. eine weitere Markierung, die an den zweiten Bindungspartner gebunden ist.

13. Die Verwendung gemäß Anspruch 12, worin die weitere Markierung eine lumineszierende Verbindung oder ein lumineszentes erzeugendes Enzym ist.

14. Die Verwendung gemäß Anspruch 12, worin der erste Bindungspartner Biotin ist.

15. Die Verwendung gemäß Anspruch 12, worin der zweite Bindungspartner gewählt ist aus der Gruppe, die im Wesentlichen aus Streptavidin, Avidin, einem Anti-Biotin-Antikörper und einem Avidin-Biotin-Komplex besteht.

16. Die Verwendung gemäß Anspruch 15, worin der zweite Bindungspartner an ein lumineszentes erzeugendes Enzym konjugiert ist.

## Revendications

1. Procédé pour déterminer rapidement l'apparition d'un syndrome de réponse inflammatoire systémique, d'une sepsie, d'une sepsie sévère ou d'un choc septique chez un sujet mammifère, ledit procédé comprenant l'étape consistant à déterminer dans un échantillon de la partie liquide du sang choisie parmi le plasma ou le sérum d'un sujet mammifère, l'existence d'enzyme oxyde nitrique synthase inductible (iNOS) en dehors de l'existence d'enzyme oxyde nitrique synthase endothéliale (eNOS) ou d'enzyme oxyde nitrique synthase neuronale (nNOS), dans lequel ladite étape utilise des moyens pour détecter l'existence d'enzyme oxyde nitrique synthase inductible (iNOS) dans la partie liquide du sang choisie parmi le plasma ou le sérum d'un sujet mammifère sans réaction croisée avec eNOS ou nNOS, comprenant un anticorps monoclonal de capture anti-iNOS et un anticorps monoclonal de détection anti-iNOS marqué, respectivement, en tant que composants de capture et de détection, dans un test ELISA en sandwich par chimioluminescence ou un test EIA en sandwich par chimioluminescence.

2. Procédé selon la revendication 1, dans lequel ledit anticorps monoclonal de capture anti-iNOS se lie à un premier site sur l'oxyde nitrique synthase inductible (iNOS) dans la partie liquide du sang d'un sujet mammifère, et ledit anticorps monoclonal de détection anti-iNOS se lie à un second site sur l'enzyme oxyde nitrique synthase inductible (iNOS) dans la partie liquide du sang d'un sujet mammifère.

3. Procédé selon la revendication 2, dans lequel ledit anticorps monoclonal de détection est marqué avec de la biotine, ou est directement conjugué à un composé luminescent ou à une enzyme produisant une luminescence.

4. Procédé selon la revendication 2, dans lequel ledit anticorps monoclonal de détection est marqué avec un premier partenaire de liaison et qui inclut en outre :
a. un second partenaire de liaison se liant audit premier partenaire de liaison ; et
b. un autre marqueur lié audit second partenaire de liaison.

5. Procédé selon la revendication 4, dans lequel ledit autre marqueur est un composé luminescent ou une enzyme produisant une luminescence.

6. Procédé selon la revendication 4, dans lequel ledit premier partenaire de liaison est la biotine.

7. Procédé selon la revendication 4, dans lequel ledit second partenaire de liaison est choisi parmi le groupe composé essentiellement de : streptavidine, avidine, un anticorps anti-biotine et un complexe avidine-biotine.

8. Procédé selon la revendication 7, dans lequel ledit second partenaire de liaison est conjugué à une enzyme produisant une luminescence.

9. Utilisation d'un appareil de dosage comprenant
des moyens pour détecter l'existence d'enzyme oxyde nitrique synthase inductible (iNOS) dans la partie liquide du sang choisie parmi le plasma ou le sérum d'un sujet mammifère sans réaction croisée avec l'enzyme oxyde nitrique synthase endothéliale (eNOS) ou l'enzyme oxyde nitrique synthase neuronale (nNOS), lesdits moyens comprenant
a. un anticorps monoclonal de capture anti-iNOS et un anticorps monoclonal de détection anti-iNOS marqué ; et
b. un format de dosage choisi parmi le groupe composé de : ELISA en sandwich par chimioluminescence et EIA en sandwich par chimioluminescence, en utilisant lesdits anticorps monoclonaux anti-iNOS, respectivement, en tant que composants de capture et de détection dans ledit dosage,
pour déterminer rapidement l'apparition d'un syndrome de réponse inflammatoire systémique, d'une sepsie, d'une sepsie sévère ou d'un choc septique chez un sujet mammifère en détectant l'existence d'enzyme oxyde nitrique synthase inductible (iNOS) dans la partie liquide du sang choisie parmi le plasma ou le sérum d'un sujet mammifère.

10. Utilisation selon la revendication 9, dans laquelle ledit anticorps monoclonal de capture anti-iNOS se lie à un premier site sur l'oxyde nitrique synthase inductible (iNOS) dans la partie liquide du sang d'un sujet mammifère, et ledit anticorps monoclonal de détection anti-iNOS se lie à un second site sur l'enzyme oxyde nitrique synthase inductible (iNOS) dans la partie liquide du sang d'un sujet mammifère.

11. Utilisation selon la revendication 10, dans laquelle ledit anticorps monoclonal de détection est marqué avec de la biotine, ou est directement conjugué à un composé luminescent ou à une enzyme produisant une luminescence.

12. Utilisation selon la revendication 10, dans laquelle ledit anticorps monoclonal de détection est marqué avec un premier partenaire de liaison et qui inclut en outre :
a. un second partenaire de liaison se liant audit premier partenaire de liaison ; et
b. un autre marqueur lié audit second partenaire de liaison.

13. Utilisation selon la revendication 12, dans laquelle ledit autre marqueur est un composé luminescent ou une enzyme produisant une luminescence.

14. Utilisation selon la revendication 12, dans laquelle ledit premier partenaire de liaison est la biotine.

15. Utilisation selon la revendication 12, dans laquelle ledit second partenaire de liaison est choisi parmi le groupe composé essentiellement de : streptavidine, avidine, un anticorps anti-biotine et un complexe avidine-biotine.

16. Utilisation selon la revendication 15, dans laquelle ledit second partenaire de liaison est conjugué à une enzyme produisant une luminescence.
